# EUROPÄISCHE PATENTANMELDUNG

(11) **EP 2 062 614 A1**
(43) Veröffentlichungstag der Anmeldung: **27.05.2009**
(21) Anmeldenummer: 07022547.9
(22) Anmeldetag: 21.11.2007
(51) Int. Cl.: A61N 2/02, A61N 1/40

(54) **Verfahren und Vorrichtung zur Impulsinduktion**

(71) Anmelder: Pulsartec S.L., 38350 Tacoronte (ES)
(72) Erfinder: Schmitt, Juan, 78628 Rottweil (DE)
(74) Vertreter: Klocke, Peter

(57) **Zusammenfassung**

Verfahren und Vorrichtung zur Erzeugung von gedämpften Entladungsströmens eines Hochspannungsenergiespeichers mit überlagerten Stromimpulsen zur Beeinflussung von biologischen Zellen. Hierbei wird mittels einer Zündkammer, welche mit einem Gas hohen Druckes befüllt ist und mittels einer Gleichspannung von mehreren tausend Volt zum Zünden gebracht wird, ein breitbandiger Entladestrom erzeugt, der dadurch entsteht, dass durch ein besonderes Impulsgebungsverfahren ein Wechselfeld bis zu gleicher Höhe der Gleichspannung aufmoduliert wird. Ein elektromagnetisches Feld wird erzeugt, welches über eine flach gewickelte Spule auf den Menschen als Resonator einwirkt und den menschlichen Zellen zusätzliche Energie zuführt, da das erzeugte elektromagnetische Feld einem natürlichen Gewitterblitz entspricht.

## Beschreibung

Die vorliegende Erfindung betrifft ein Verfahren zur Erzeugung von gedämpften Entladungsströmungen eines Hochspannungsenergiespeichers mit überlagerten Stromimpulsen zur Beeinflussung von biologischen Zellen sowie eine Vorrichtung zur Durchführung des Verfahrens.

Aus der WO 2005/001496 A1 und der WO 94/01176 A1 ist ein derartiges Verfahren und Vorrichtung bekannt. Im Wesentlichen wird dort über einen Hochspannungstransformator ein Energiespeicher aufgeladen, der dann über eine Funkenstrecke in einem gasgefüllten Raum entladen wird. Der Entladestrom wird auf eine besonders ausgestaltete Behandlungssonde gegeben, um die von dem Strom in der Behandlungssonde induzierten Schwingungen auf einen Menschen zu übertragen. Bei der Entladung des Energiespeichers entsteht eine gedämpfte Sinusschwingung hoher Frequenz. Die Zündung des Lichtbogens zur Entladung des Energiespeichers erfolgt mittels einer Thyratron-Röhre, die an den maximalen Amplituden der Trägerwelle im erzeugten Plasma einen Impuls aufsetzt. Bei einem derartigen Impuls handelt es sich bei größerer zeitlicher Auflösung wiederum um eine gedämpfte Sinusschwingung mit höherer Frequenz.

Abhängig von dem verwendeten Gas in der Entladekammer werden unterschiedliche Entladespitzen erzeugt.

Hintergrund dieses Verfahrens und dieser Vorrichtung ist die Erkenntnis, dass mittels der über die Behandlungssonde induzierten Schwingungen die Zellen im menschlichen Körper positiv beeinflusst werden können. Diese Erkenntnis basiert auf Forschungen, die gezeigt haben, dass natürliche Blitzentladungen eine wohltuende und aktivierende Wechselwirkung auf alle lebendigen Systeme, wie Menschen, Tiere und auch Pflanzen, ausüben, also auch die Biosphäre vollständig mit einschließen. Im Nahfeld solcher hoch energetischer Entladungen wurden auch verschiedentlich so genannte Spontanheilungen beobachtet.

Das bekannte Verfahren und die Vorrichtung erzeugen jedoch, wie die Diagramme in den vorstehend erwähnten Patentanmeldungen zeigen, insbesondere in der WO 2005/001496 A1, keinen Verlauf, der nur annähernd einer Entladung eines Gewitterblitzes entspricht. Darüber hinaus weist das Gerät insofern bauliche Nachteile auf, als beim Betrieb eines Thyratron Röntgenstrahlungen entstehen, die besondere Abschirmungen und Schutzmaßnahmen nötig machen. Bauliche Nachteile dieses bekannten Geräts sind außer den verursachten Röntgenstrahlen auch das in der offenen Zündkammer erzeugte Ozon. Dieses Ozon muss entweder durch einen am Gehäuse angebrachten Ozonfilter gefiltert oder durch einen Abluftschlauch ins Freie entsorgt werden.

Der vorliegenden Erfindung liegt daher die Aufgabe zugrunde, ein Verfahren und eine Vorrichtung vorzuschlagen, mit der zeitlich gesteuerte Entladungsimpulse gebildet werden können, die einem natürlichen Gewitterblitz möglichst nahe kommen, und die dabei entstehende Feldenergie in den menschlichen Körper einzubringen.

Diese Aufgabe wird erfindungsgemäß mit den Merkmalen des Verfahrensanspruchs und des Vorrichtungsanspruchs gelöst. Weitere vorteilhafte Ausgestaltungen sind den jeweiligen rückbezogenen Unteransprüchen zu entnehmen.

Danach wird der Hochspannungsenergiespeicher wiederholt mit Pausen durch breitbandige mit einer bis in den Nanosekundenbereich reichenden Periodendauer Entladeströme entladen und anschließend werden aus den Entladeströmen elektromagnetische Wellen, insbesondere Longitudinalwellen, erzeugt.

Durch diese besondere Breitbandigkeit des Frequenzspektrums von einigen Kilohertz bis zu mehreren hundert Megahertz werden die natürlichen atmosphärischen Entladungen bei Blitzen besonders gut nachgebildet. Durch die Breitbandigkeit des Frequenzspektrums können alle Zellen eines Organismus gemäß ihren unterschiedlichen spezifischen Eigenfrequenzen in Resonanz versetzt und somit energetisiert, regeneriert bzw. sogar wieder belebt werden.

Dies basiert darauf, dass Wachstum und Funktion der Zellen durch elektromagnetische Schwingungen gesteuert werden. Störungen dieser Information führen zu Zellschäden und damit zu Krankheiten, die in der Folge zu einem messbaren Mangel an Energie in den Zellen führen. Eine gesunde Zelle weist in der Regel eine Spannung an ihrer Membran auf, welche ungefähr -70 bis -80 mV beträgt. Bricht diese Spannung zusammen, so treten in einem ersten Schritt beispielsweise entzündliche Prozesse auf. Versucht im weiteren Verlauf die Zelle sich zu retten, so ist ein Notprogramm erforderlich, in dem sie anweist, sich zu teilen und zu wachsen. Dies kann als der Ursprung der meisten Tumorbildungen angesehen werden. Diesen Energiemangelkrankheiten kann mittels externer "elektromagnetischer" Impulse entgegengewirkt werden, da diese in der Lage sind, aufgrund ihrer Struktur, Energie den Zellen zuzuführen. Dazu müssen sowohl die Resonanzbedingungen als auch die Form der Impulse stimmen. Da jede Zelle oder auch jeder Zellverbund seine eigenen Informationen und damit Schwingungen besitzt, kann nur durch ein sehr breitbandiges Frequenzgemisch auch jede Zelle erreicht werden, denn die Regeneration der Membranspannung, welche dem Austausch von Informationen und Nährstoffen dient, ist nur durch exakte Resonanzbedingungen sowie der Art und/oder der Form der Impulse wiederherstellbar.

Gemäß dem Verfahren werden insbesondere Longitudinalwellen erzeugt, um damit die Zellen zu beeinflussen. Jeder elektromagnetische Impuls besteht grundsätzlich aus zwei Komponenten, und zwar aus einer transversalen Welle (auch Hertz'sche Welle genannt) und einer Longitudinalwelle (auch Tesla Welle genannt). Bekanntlich bewegt sich die Transversalwelle senkrecht zur Ausbreitungsrichtung, so dass sie damit von ihr entkoppelt erscheint. Im Falle von Hochfrequenzen wirkt hier der biologische Körper wie ein Faradayscher Käfig. Die Welle kann somit die Haut nicht oder kaum durchdringen, so dass von hier auch keine biologische Wirkung zu erwarten ist. Longitudinalwellen, wie beispielsweise auch eine Schallwelle, sind Stoßwellen, die sich in Längsrichtung fortbewegen und dadurch auch Materialien durchdringen können (vgl. Schall vs. Licht). Grundlegende Erkenntnisse hierzu basieren auf den Entdeckungen und Entwicklungen von Nicola Tesla. Ausführliche Erläuterungen dazu sind auch den Publikationen von Professor Konrad Meyl, elektromagnetische Umweltverträglichkeit 1 bis 3 zu entnehmen. Die nachgewiesene Besonderheit bei den Longitudinalwellen ist die, dass eine Energieübertragung nur dann stattfindet, wenn der Sender und der Empfänger aufeinander abgestimmt, in Resonanz sind. Da die Resonanzbedingung bei den verschiedenen Zellen entsprechend ihres augenblicklichen Zustandes unterschiedlich ist, ist die vorstehend erwähnte Breitbandigkeit erforderlich. Es wird damit auch erreicht, dass eine Energieübertragung nur dann stattfindet, wenn diese erforderlich ist, d.h., die Resonanzbedingung vorliegt. Diese wird bei sich ändernden Zellen sich auch entsprechend verändern, so dass zu erwarten ist, dass keine schädlichen Wirkungen auftreten. Aus diesem Grunde ist es wichtig, dass insbesondere Longitudinalwellen und nur ein physikalisch bedingter kleiner Anteil an Transversalwellen erzeugt wird. Durch die Resonanz in den Zellen, Mitochondrien und Atomen wird so für einen messbaren Energiezuwachs und damit einhergehend eine Aufhellung des Gemüts durch weniger Leiden gesorgt. Die Messung kann zum Beispiel in Form einer Spannungserhöhung an den Zellmembranen oder durch Photonenresonanz mit entsprechenden Lichtverstärkern nach Prof. Albert Popp erfolgen.

Damit das neuronale System eine ausreichende Erholungszeit der Nervenzellen nach einer Reizübertragung (Refraktärzeit) hat, wird gemäß dem erfindungsgemäßen Verfahren der Energiespeicher maximal drei mal pro Sekunde, vorzugsweise ein mal pro Sekunde entladen.

Zur Durchführung des Verfahrens wird eine Gleichspannungserzeugungseinrichtung verwendet, deren Ausgang mit mindestens einer Hochspannungsimpulserzeugungseinrichtung, welche eine Hochspannung mit überlagerten Hochspannungsimpulsen generiert, verbunden ist. Im Anschluss an die Hochspannungsimpulserzeugungseinrichtung befindet sich ein Energiespeicher der mittels der Hochspannung aufgeladen wird. Des weiteren weist die Vorrichtung eine gasgefüllte Zündkammer zum Erzeugen eines Lichtbogens beim Überschreiten der Ionisationsspannung durch die Spannung an dem Energiespeicher, und eine Behandlungssonde im Anschluss an die Zündkammer zur Übertragung der durch die Zündungen ausgelösten Stromentladungsimpulse auf biologische Zellen auf.

Bei der Gleichspannungserzeugungseinrichtung ist es zweckmäßig, ein getaktetes Netzteil zu verwenden, um elektromagnetische schädliche Schwingungen (50 Hertz-Netzfrequenz) die in die Schaltung eingreifen, zu verhindern. Dies kann auch durch konventionelle Netzteile mit Netztrafo und Gleichrichter verhindert werden, die mit bekannten umfangreichen Filter- und Schutzmaßnahmen versehen sind. Die Regelbarkeit der Gleichspannung am Ausgang des Netzteils ist für die Beeinflussung der Aufladezeit des Energiespeichers von Bedeutung.

Vorteilhafterweise weist die Hochspannungsimpulserzeugungseinrichtung eine Hochspannungszündeinrichtung und einen ersten Hochspannungstransformator auf, wobei die Zündeinrichtung mittels eines elektronischen Leistungsschalters, beispielsweise Thyristors gesteuert, in vorgebbaren Zeitabständen einen zweiten Hochspannungstransformator speist. Hierzu kann als Hochspannungszündeinrichtung eine Einrichtung verwendet werden, die auf bekannten Zündeinrichtungen für Verbrennungsmotoren basiert. Die Hochspannungsimpulserzeugungseinrichtung wird durch die geregelte Gleichspannung von der Gleichspannungserzeugungseinrichtung gespeist. Die am Ausgang der Hochspannungsimpulserzeugungseinheit erzeugte

Hochspannung ist eine Gleichspannung, welche durch eine bis zu gleicher Spannungshöhe überlagerte Impulsspannung den Hochspannungsenergiespeicher, beispielsweise Kondensator, lädt. Der elektronische Leistungsschalter kann mittels eines Frequenzgenerators mit regelbarer Frequenz und veränderbaren Pulsbreiten gesteuert werden.

Gemäß einer bevorzugten Ausbildung der Hochspannungsimpulserzeugungseinrichtung weist die Hochspannungszündeinrichtung Mittel zur leistungsarmen Erzeugung einer Gleichspannung am Ausgang der Hochspannungszündeinrichtung auf. Hierzu wird ein Zündladekondensator von einem ersten Hochspannungstransformators aufgeladen. Ein als Übertrager wirkender zweiter Transformator in der Hochspannungszündeinrichtung wird mit dem Entladestrom des Zündladekondensators und nach dem Entladen des Zündladekondensators mit dem durch den Entladevorgang in dem Hochspannungstransformator aufgebauten Magnetfeld erzeugten Induktionsstrom beaufschlagt. Durch diese Maßnahme wirkt der zweite Transformator beim Entladen des Zündladekondensators und danach beim Zusammenbrechen des Magnetfeldes, wenn der Zündladekondensator entladen ist. Infolge dessen kann ungefähr die doppelte Energie übertragen werden, ohne den Hochspannungstransformator zu überlasten. Bei den bekannten Hochspannungszündeinrichtungen wird nur der Teil benutzt, der beim Entladen des Zündladekondensators entsteht.

Die der Hochspannung überlagerten Hochspannungsimpulse weisen bis zu etwa der gleichen Höhe, vorzugsweise bis zu einer Gesamtamplitude von 30 kV, auf.

Vorteilhafterweise weist die Zündkammer ein Edelgas, vorzugsweise Xenon, auf, welches besonders bevorzugt unter einem Druck von bis zu 2 bis 3 MPa steht. Als Zündkammer kann beispielsweise eine übliche Xenonlampe, wie sie für Leuchttürme oder Skybeamer eingesetzt wird, verwendet werden. Das Xenonlicht kommt dem natürlichen Sonnenlicht am nächsten, das heißt, dass bei weißem Licht alle Farben und damit verbunden alle Spektralfrequenzen gleichermaßen vorhanden sind., was sich bei der Erfindung als vorteilhaft erwiesen hat Andere Edelgase sind farblastig und heben dadurch nur einen bestimmten Frequenzbereich hervor

Grundsätzlich können bei der Behandlungssonde alle als induktionsfrei (oder induktionsarm) geltenden Spulenformen benutzt werden, also z.B. auch eine Möbiusschleife. Dies ist insofern wichtig, als hier der Transversalanteil der Welle gegen Null tendiert (theoretisch Null, praktisch jedoch kaum 100% zu verwirklichen). Zweckmäßigerweise weist die Behandlungssonde daher eine Flachspule mit parallel geführten Anschlussleitungen an die Zündkammer auf, wobei die Flachspule vorzugsweise 7 Windungen bei einer Kabelgesamtlänge der Flachspule mit Anschlusskabeln von 5,5 m ± 2 % aufweist. Mittels dieser Behandlungssonde, erfolgen die Umwandlungen der Stromimpulse in Longitudinalwellen, wie bereits bei Tesla beschrieben. Es hat sich allerdings gezeigt, dass besonders gute Ergebnisse bei einer Flachspule mit den vorstehend angegebenen Abmessungen erzielt werden kann. Dabei kann die Flachspule einfach oder als bifilare Flachspule ausgebildet sein. Grundsätzlich können auch gleichzeitig mehrere parallel geschaltete Flachspulen verwendet werden. Im Falle einer Bifilarflachspule weist diese ebenfalls 7 Windungen und die vorgegebene Kabelgesamtlänge auf. Vorteilhafterweise sind die Innenleiter der Behandlungssonde als verdrillte Einzelleiter, zweckmäßigerweise stark verdrillten, Einzelleitern ausgebildet. Es hat sich nämlich gezeigt, dass mit der Erhöhung der Verdrillung ein Verstärkungseffekt gegenüber einer geringeren Verdrillung entsteht, in dem das durch die Verdrillung im Inneren des Kabels fließende Magnetfeld ein wirbelndes Plasma-Magnetfeld erzeugt, das durch den Drall die elektrodynamische Wirkung beschleunigt und vermehrt.

Mit der vorstehend beschriebenen Einrichtung wurden verschiedene Personen mit unterschiedlichen Leiden behandelt. Dabei hat sich gezeigt, dass nach ein- oder mehrmaligen Behandlungen mit einer Dauer von cirka 15 Minuten, wobei die Anzahl der Behandlungen von dem Grad der Beschwerden abhing, folgenden Beschwerden abgeholfen werden konnten:
Frau, Alter 34, klagte über Verspannungen der Rückenmuskulatur. Nach einer einmaligen Behandlung von cirka 10 Minuten war eine spürbare und deutliche Verbesserung der Beschwerden zu erkennen.
Mann, Alter 49, hatte starke Gelenkschmerzen im Ellbogen sowie Beschwerden im Rücken. Nach zwei Behandlungen in einem Zeitraum von zwei Wochen waren die Rückenbeschwerden völlig verschwunden und am Ellbogen noch leichte Beschwerden vorhanden.
Mann, Alter 50, hatte Verspannungen im rechten Unterarm und Gelenk sowie Rückenprobleme. Nach zwei Behandlungen im Zeitraum von 14 Tagen waren die Rückenprobleme verschwunden. Auch am Arm konnte eine wesentliche Verbesserung festgestellt werden. Der Arm war schmerzfrei.
Frau, Alter 38, mit einer Zahnfleischentzündung, konnte nach einer einmaligen Behandlung von cirka 10 Minuten erfolgreich geholfen werden, wohin gegen vorangegangene Spülungen und professionelle Zahnreinigungen keinen Erfolg gezeigt hatten.
Mann, Alter 44, hatte sich nach einem Sturz vom Fahrrad ein blaues Auge zugezogen. Nach zwei Behandlungen über cirka 10 Minuten in einem Zeitraum von sechs Tagen war das Hämatom nach sechs Tagen vollkommen verschwunden und keine Spuren mehr sichtbar.
Mann, Alter 26, klagte über starke Knöchelbeschwerden, die ein Auftreten nicht mehr möglich machten. Nach einer einmaligen Behandlung von cirka 10 Minuten konnte der Patient eine sofortige Verbesserung feststellen und am nächsten Morgen absolut beschwerdefrei auftreten.
Mittels des erfindungsgemäßen Verfahrens und der Vorrichtung kann somit eine Möglichkeit bereitgestellt werden, mit der die natürlichen Bedingungen bei einer atmosphärischen Entladung möglichst weitgehend nachgebildet und Longitudinalwellen erzeugt werden können, die eine außerordentliche therapeutische Wirkung zeigen. Das Gerät ist von der Größe eines Werkzeugkoffers und wiegt cirka 20 kg, so dass es leicht zu der zu behandelnden Person gebracht werden kann.
Weitere Merkmale der Erfindung ergeben sich aus der folgenden Beschreibung eines Ausführungsbeispiels in Verbindung mit den Ansprüchen und den Zeichnungen. Die einzelnen Merkmale können hier für sich oder zu mehreren bei Ausführungsformen der Erfindung verwirklicht sein. Es stellen dar:
   - Figur 1: eine Prinzipskizze der Vorrichtung;
   - Figur 2: ein Blockschaltbild der erfindungsgemäßen Vorrichtung;
   - Figur 3: die Detaildarstellung einer handelsüblichen Hochspannungszündeinrichtung in verschiedenen Schaltphasen (Figuren 3a, 3b); eine besondere Ausführungsform einer besonders abgestimmten Hochspannungszündeinrichtung (Figur 3c);
   - Figur 4: zwei Ausführungsformen der Behandlungssonde; einfach (Figur 4a) und bifilar (Figur 4b)
   - Figur 5: Vergleich von im Zentrum der Spule der Behandlungssonde gemäß Figur 4 gemessene "Impulsverläufe" nach dem Stand

   - Figur 6 und 7: der Technik (Figur 5a, Figur 5b), gemäß der Erfindung (Figur 5c), sowie bei natürlichen Gewittern (5d); Oszillogramme eines Impulsverlaufs mit immer größer werdender Auflösung.

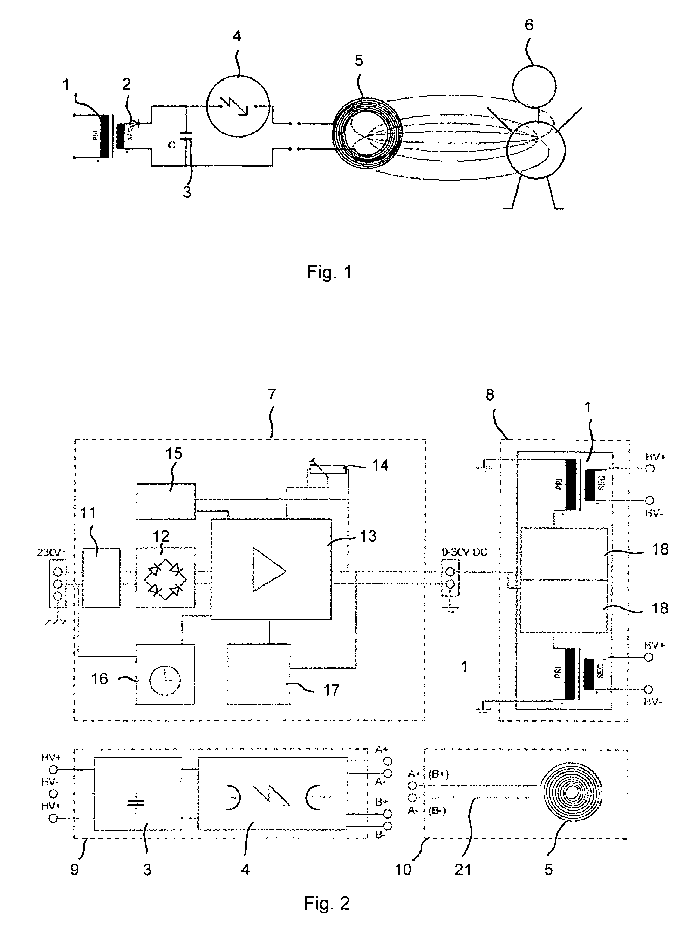

Figur 1 zeigt das an sich allgemein bekannte grundsätzliche Prinzip für den Aufbau einer derartigen Vorrichtung, wie es auch im Stand der Technik benutzt wird. Mittels eines Hochspannungstransformators 1 wird über eine Diode 2 ein Energiespeicher 3 in Form eines Kondensators aufgeladen. Dieser wird über eine Funkenstrecke 4 entladen und der Ladestrom über eine Spule 5 geführt. Die Spule 5 erzeugt eine elektromagnetische Welle, die in Resonanz mit dem biologischen System, hier Mensch 6, tritt. Im vorliegenden Fall wird eine Kurzbogenlampe, die mit einem Gas mit hohem Druck befüllt ist, als Funkenstrecke, nachfolgend als Zündkammer bezeichnet, verwendet. Selbstverständlich können auch andere geeignete gasgefüllte Zündkammern eingesetzt werden, die beispielsweise auch einen variablen Abstand zwischen den Elektroden erlauben und deren Elektrodenform so ausgebildet ist, dass sie bei häufigen pulsartigen Entladungen eine entsprechende Standzeit aufweisen. Die in dem Ausführungsbeispiel verwendete Kurzbogenlampe weist einen Elektrodenabstand von cirka 7 mm auf und ist mit Xenon gefüllt, das im kalten Zustand einen Druck von 2 bis 3 MPa aufweist. Die hier verwendete Kurzbogenlampe ist zweckentfremdet, da sie eigentlich für einen Dauerbetrieb gedacht ist.

Die Kurzbogenlampe als Zündkammer 4 wird mittels einer Gleichspannung von mehreren tausend Volt zum Zünden gebracht. Dabei wird durch ein besonderes Impulsgebungsverfahren ein Wechselfeld bis zu gleicher Höhe der Gleichspannung aufmoduliert, welches im Zündmoment zum Tragen kommt. Bei der Entladung wird ein elektromagnetisches Feld erzeugt, welches über die flachgewickelte Spule 5 auf den Menschen 6 als Resonator einwirkt und den menschlichen Zellen im Resonanzfall Energie zuführt. Entsprechend sind auch die Feldlinien zwischen der Spule 5 und dem Menschen 6 dargestellt. Wichtig bei dieser Anordnung ist die Resonanzfrequenz, welche durch die baulich bedingte Rest-Induktivität L der Spule 5 und der Kapazität C des Energiespeichers 3 gemäß in der allgemein bekannten Schwingkreisformel bestimmt wird. Durch das besondere Impulsgebungsverfahren wird auf die Resonanz-Trägerwelle eine Reihe von unterschiedlichen Frequenzen aufmoduliert, die nach der Entladung in Form von einer stark gedämpften sinusförmigen Wechselspannung multipler Frequenz im Zentrum der Spule gemessen werden kann. Durch die Ausbildung der Spule 5 als Flachspule werden von dieser überwiegend Longitudinalwellen abgegeben, die auf den Menschen 6 treffen.

Figur 2 zeigt das Blockschaltbild der erfindungsgemäßen Vorrichtung bestehend aus einem Schaltnetzteil 7, einer Hochspannungsimpulserzeugungseinrichtung 8, einer Hochspannungszentraleinheit 9 und einer Behandlungssonde 10.

Bei dem Schaltnetzteil 7 handelt es sich um ein nach bekannten Prinzipien aufgebautes Netzteil, welches eine Gleichspannung von 5 bis 30 Volt abgibt, die möglichst oberwellenfrei variiert werden kann. Über die Einstellungen an dem Schaltnetzteil besteht die Möglichkeit, die Anzahl der Entladungen des Hochspannungsenergiespeichers 3 durch die Zündkammer 4 zu bestimmen. Dabei ist es wichtig, dass ein neuer Aufladevorgang und damit eine neue Entladung erst dann erfolgt, wenn der Hochspannungsenergiespeicher 3 möglichst vollständig entladen ist, damit beim Zünden und dem dabei entstehenden Entladestrom die vollständige maximale Spannungsdifferenz ausgenutzt wird. Das Schaltnetzteil 7 weist in bekannter Art und Weise einen Netzfilter 11, einen Gleichrichter 12, einen Schaltregler 13, eine Gleichspannungsregelung 14 und eine Einrichtung 15 für die Bestimmung der Wechselintervalle der als Zündkammer 4 verwendeten Lampe sowie eine Zeitschaltuhr 16 und eine Regelung für Überlast und Temperatur 17 auf. In der Zeitschaltuhr 16 wird die Behandlungszeit eingestellt.

Die Gleichspannung wird an die Hochspannungsimpulserzeugungseinrichtung 8 angelegt, die in dem Ausführungsbeispiel zwei Hochspannungszündeinrichtungen 18 und zwei darauf folgende Hochspannungstransformatoren 19 aufweist, um die hohe geforderte Abgabeleistung zu ermöglichen. Am Ausgang der Hochspannungsimpulserzeugungseinrichtung liegt potentialfrei die Hochspannung an, die dem Energiespeicher 3 in der Hochspannungszentraleinheit 9 zugeführt wird. Die am Ausgang der Hochspannungsimpulserzeugungseinrichtung anliegende Hochspannung ist eine Gleichspannung, welche durch eine überlagerte Impulsspannung den Energiespeicher 3 lädt. Diese Hochspannung erzeugt die Zündung in der Hochspannungszündkammer 4 und moduliert auf den normalerweise in der üblichen Form als stark gedämpften sinusförmigen abnehmenden Entladestrom zusätzliche Impulse, die die Besonderheit der Erfindung und die Auswirkungen auf den Menschen bzw. die biologischen Zellen ausmachen. In dem Ausführungsbeispiel besteht der Energiespeicher 3 aus Hochspannungskondensatoren mit einer Gesamtkapazität von cirka 70-90 nF. Die Größe des Energiespeichers 3 beeinflusst bekanntlich den gesamten Entladevorgang.

Wie schon erwähnt, besteht die Hochspannungszündkammer 4 in dem Ausführungsbeispiel aus einer Funkenstrecke, die mit einem Gas und hohem Druck umgeben ist. Hierbei kann es sich um Xenon oder um ein anderes Edelgas handeln, wobei für das Prinzip der Erfindung es nicht erforderlich ist, dass verschiedene Gase für die Erzeugung von verschiedenen Impulsen eingesetzt werden müssen, wie dies beim Stand der Technik üblich ist.

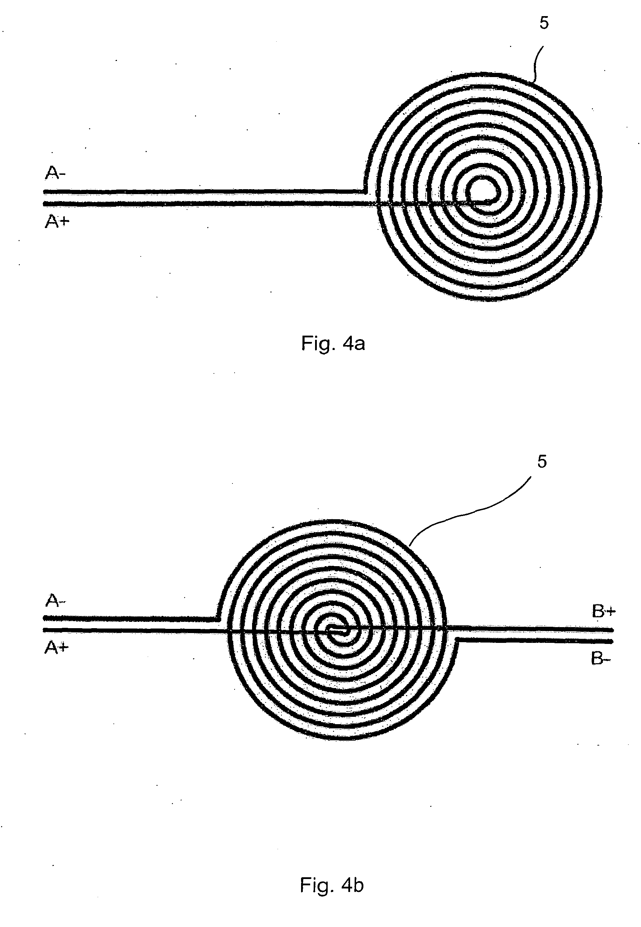

Im Anschluss an die Hochspannungszentraleinheit 9 können eine oder zwei Behandlungssonden 10 wie in Figur 4a dargestellt, oder alternativ eine bifilar gewickelte Behandlungssonde, wie in Figur 4b dargestellt, angeschlossen werden. Die Behandlungssonden 10 sind mit einem hochspannungsfesten Kabel hergestellt, wobei, wie allgemein bekannt, der Stromfluss von außen nach innen erfolgen muss. Da die Länge des Kabels, wie vorstehend erwähnt, die Resonanzfrequenz beeinflusst, hat sich ein Optimum auch hinsichtlich der Wirkung beim Menschen bei 7 Windungen gezeigt, wobei die Gesamtlänge des in der Behandlungssonde verwendeten Kabels 5,5m ± 2 % beträgt.

Zur Steuerung der Hochspannungsimpulserzeugungseinrichtung 8 wird jede Hochspannungszündeinheit 18 mit einer regelbaren Gleichspannung von 5 bis 30 Volt versorgt. Abhängig von der Gleichspannung werden von der Hochspannungszündeinheit Hochspannungsimpulse erzeugt. Alternativ könnte auch statt der veränderlichen Gleichspannung des Schaltnetzteils eine feste Gleichspannung angelegt und die Frequenzen der Hochspannungszündeinheit 18 verändert werden. Die Hochspannungszündeinheit 18 kann eine allgemein übliche und bekannte Schaltungsanordnung wie sie zur Steuerung von Verbrennungsmotoren verwendet wird, beinhalten. Diese sind handelsüblich unter der Bezeichnung Hochspannungskondensatorzündung (HKZ) erhältlich und werden auch als Thyristorzündung bezeichnet. Im Ausführungsbeispiel wird eine speziell für diesen Zweck entwickelte Zündeinheit zur Optimierung verwendet.

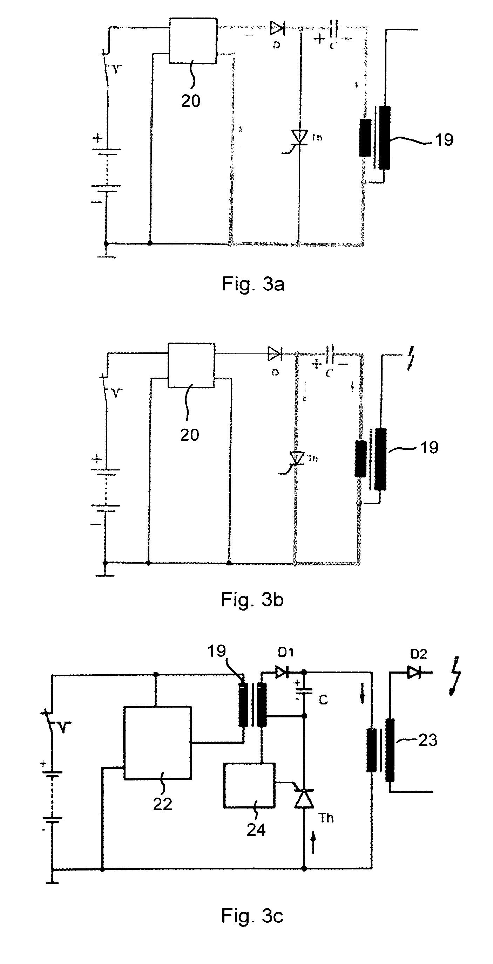

In Figur 3 ist diese allgemein bekannte Schaltung beim Aufladen (Figur 3a) und beim Entladen (Figur 3b) schematisch (unter weglassen von für den betrachteten Vorgang nicht relevanten elektronischen Bauteilen, die dem Fachmann bekannt sind) dargestellt. Gemäß Figur 3a wird unter Verwendung einer nicht näher bezeichneten Steuerschaltung 20 der Speicherkondensator C auf eine Spannung aufgeladen und im Zündzeitpunkt durch Schließen des elektronischen Leistungsschalters, in diesem Fall eines Thyristors Th, über die Primärwicklung eines Hochspannungstransformators 19 als Zündtransformator entladen. Da der Kondensator C und die Primärwicklung des Hochspannungstransformators einen Schwingkreis bilden, entstehen gedämpfte Schwingungen von Strom und Spannung. Figur 3a zeigt die Energiespeicherung durch Aufladen des Kondensators C. Der Thyristor Th sperrt den Stromdurchgang. Gemäß Figur 3b erfolgt die Übertragung der gespeicherten Zündenergie auf den Zündkreis durch Entladen des Kondensators C. Der Thyristor Th hat durchgeschaltet. Die am Ausgang des Hochspannungstransformators 19 abgegebene Spannung wird danach auf den bzw. die Hochspannungstransformatoren 1 gegeben. Die um Ansgang abgegeben Spannung muss noch durch einen nicht dargestellten Gleichrichter gleichgerichtet werden.

Die Figur 3c zeigt eine prinzipielle funktionsmäßige Darstellung einer bevorzugten Schaltung in der Hochspannungszündeinheit 18. Diese enthält eine Zündschaltung 22 sowie einen Hochspannungstransformator 19, dessen Primärwicklung einerseits mit der Spannungsquelle und andererseits mit der Zündschaltung 22 verbunden ist. Die Zündschaltung 22 wirkt dabei derart, dass bei einem anliegenden Eingangssignal über den Hochspannungstransformator 19 und über die Diode D1 der Kondensator C aufgeladen wird. Dabei ist die Zündschaltung 22 so ausgelegt, dass der Stromfluss durch die Primärwicklung des Hochspannungstransformators 19 nach einer gewissen Zeit abgeschaltet wird und die in dem Hochspannungstransformator 19 gespeicherte magnetische Energie über die Diode D1 den Kondensator C auflädt. Im Gegensatz zu den bekannten vorstehend erwähnten Thyristorzündungen findet keine laufende Aufladung über den Hochspannungstransformator 19 statt. Bis zur nächsten positiven Flanke eines an die Zündschaltung 22 angelegten Eingangssignals fließt lediglich ein geringer Strom in der Zündschaltung 22. Die Einschaltung durch eine positive Flanke eines Eingangssignals bewirkt auch die Zündung des Thyristors Th durch die Steuerschaltung 24, so dass der Kondensator C über die Primärwicklung des als Übertrager wirkenden Transformators 23, wie auch im Zusammenhang mit der Figur 3b beschrieben, entladen wird. Ist der Zündkondensator C entladen, ist das Magnetfeld in dem Transformator 23 noch durch den Entladestrom des Zündkondensators C aufgebaut. Da keine Spannung mehr anliegt, sperrt der Thyristor Th und das im Transformator enthaltene Restmagnetfeld bewirkt einen Induktionsstrom auf der Sekundärseite des Transformators 23 durch die Diode D2. Der Lichtbogen in der Zündkammer 4 brennt so lange als Gleichstromlichtbogen weiter, bis nahezu alle noch im Magnetfeld des Transformators 23 enthaltene Energie aufgebraucht ist. Da die Entladezeit des Zündkondensators C wesentlich kürzer ist als die Zeit, die benötigt wird, um das Magnetfeld in dem Hochspannungstransformator 19 aufzubauen, ist der Zündkondensator C bereits entladen, wenn der Hochspannungstransformator 19 abgeschaltet und die Wiederaufladung des Zündkondensators C durch die in dem Hochspannungstransformator 19 gespeicherte Energie beginnt. Bei diesem Vorgang arbeitet der Transformator 23 zuerst als Flusswandler (beim Entladen des Zündkondensators C), danach als Sperrwandler (Zusammenbrechen des Magnetfeldes). Deswegen kann auch ungefähr die doppelte Energie übertragen werden, ohne den Hochspannungstransformator zu überlasten. Bei der bekannten Thyristorzündung arbeitet die Zündspule nur als Sperrwandler und der Strom durch die Primärspule wechselt die Richtung zwischen den beiden Zuständen. Bei der Schaltung gemäß Figur 3c fließt der Strom durch den Transformator 23 immer in die gleiche Richtung. Die Diode D2 auf der Sekundärseite des Transformators 23 besorgt die notwendige Gleichrichtung für die nächste Stufe.

Die Ladeschaltung 22 ermöglicht den Einbau zusätzlicher Regelungen, beispielsweise derart, dass Betriebsspannungen in einem Bereich von cirka 3,5 V bis cirka 18 V nahezu keinen Einfluss auf die Zündleistungen haben. Des weiteren kann bei ansteigenden Temperaturen die Zündleistung konstant gehalten werden. Darüber hinaus erlaubt die Zündfunkenerzeugung die Verwendung von nahezu jedem beliebigen Hochspannungstransformator, wobei jedoch Hochspannungstransformationen mit hohem Übersetzungsverhältnis die Erhöhung des Abstandes zwischen den Zündelektroden in der Zündkammer 4 gestatten. Des weiteren kann die Nachregelung der Zündleistung vorgesehen werden, wenn sich der Abstand der Elektroden durch Abnutzung vergrößert, so dass stets für eine gleichbleibende Zündleistung gesorgt ist.

Die vorstehend beschriebene Schaltungsanordnung der Hochspannungszündeinheit 18 kann auch als selbstständige Zündeinheit beispielsweise für die Handsteuerung von Verbrennungsmotoren verwendet werden. Sie ist in ihrem Grundaufbau nicht nur für die in diesem Zusammenhang beschriebene Vorrichtung zur Erzeugung von gedämpften Entladungsströmen einsetzbar.

Die Besonderheit dieses Aufbaus liegt darin, dass durch zwei hintereinander geschaltete Hochspannungstransformatoren, eine Hochspannung mit zusätzlich überlagerten Impulsen erzeugt wird, die dann bei der Zündung in der Zündkammer 4 breitbandige Entladeströme erzeugen, die weitgehend an die natürlichen Entladungen bei Blitzen angenähert sind. Die Zündung des Thyristors erhält dieser durch einen vorgeschalteten Impulsgenerator 18 mit einer Frequenz von ca. 200 Hz bis 600 Hz. Bei dem Ausführungsbeispiel erfolgt die Entladung des Energiespeichers 3 über eine Zeitdauer von 3 bis 4 Millisekunden.

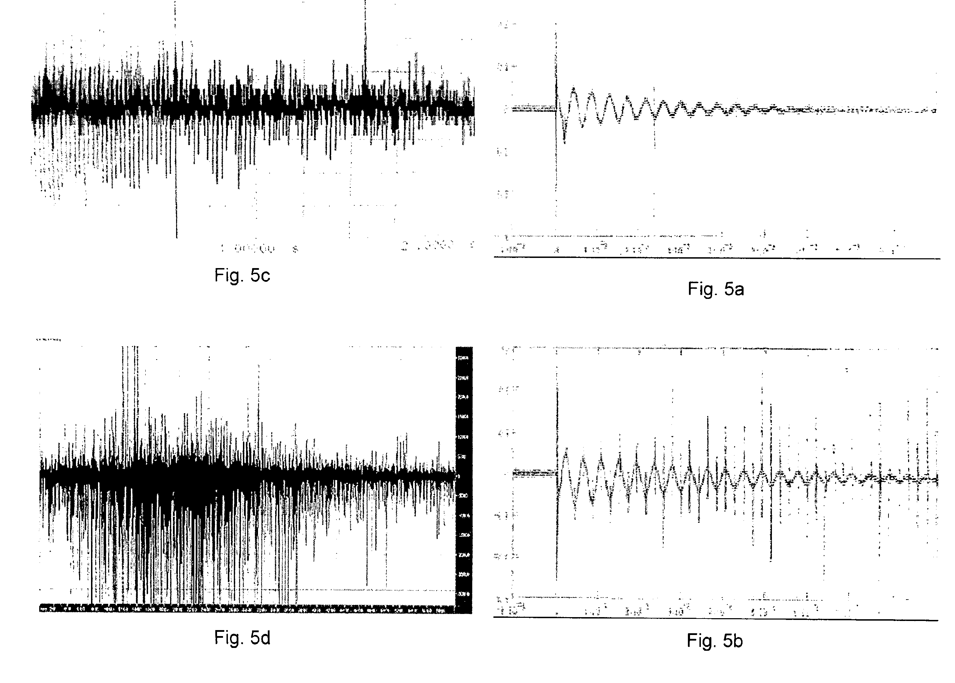

Figur 5 zeigt in einer Gegenüberstellung die Stromverläufe von bekannten Vorrichtungen in den Figuren 5a und 5b und der erfindungsgemäßen Vorrichtung in Figur 5c bei gleicher Auflösung. Bei einem bekannten, auf den Markt erhältlichen Gerät gemäß Figur 5a, bei dem nur ein Thyristormodul als Hochspannungsschalter dient, so wie es in Figur 3 im Zusammenhang mit der hier verwendeten Hochspannungszündeinrichtung beschrieben ist, ist hier nur ein Zündimpuls gefolgt von einer abschwellenden und somit gedämpften Sinusschwingung ersichtlich. Die beiden einzelnen Spitzen darunter sind eher Zufalls- oder Fehlzündungen. Man sieht hier deutlich, dass außer der Trägerwelle keine weiteren nutzbaren Frequenzen zur Verfügung stehen. Dies rührt daher, dass kein Blitz erzeugt wird, sondern einfach über einen elektronischen Thyristorschalter, die an den Hochspannungskondensatoren anliegende Hochspannung kurzgeschlossen wird, was den Impuls für die Trägerwelle auslöst. Bei dem Stromverlauf gemäß Figur 5b handelt es sich um den in der WO 2005/0014996 A1 dargestellten Stromverlauf. Hier befinden sich zusätzliche Impulse an den maximalen Amplituden der Trägerwelle, bedingt durch den Aufbau mittels eines Thyratrons. Der Verlauf der Welle wird durch das verwendete Gas beeinflusst und gezielt ausgenutzt. In einer größeren Auflösung sind die einzelnen Spitzen ebenfalls wieder als sinusförmige Schwingungen mit höher Frequenz darstellbar. In Figur 5c ist der Stromverlauf gemäß der Erfindung ersichtlich, der eine Vielzahl von zusätzlichen Entladungsspitzen aufweist, die der natürlichen Entladung wesentlich mehr gleichen.

Figur 5d zeigt die Entladung bei natürlichen Gewittern. Aus dem Vergleich wird offenbar, dass die nach der Erfindung erzeugten Spannungsverläufe denen eines Gewitters am nächsten kommen.

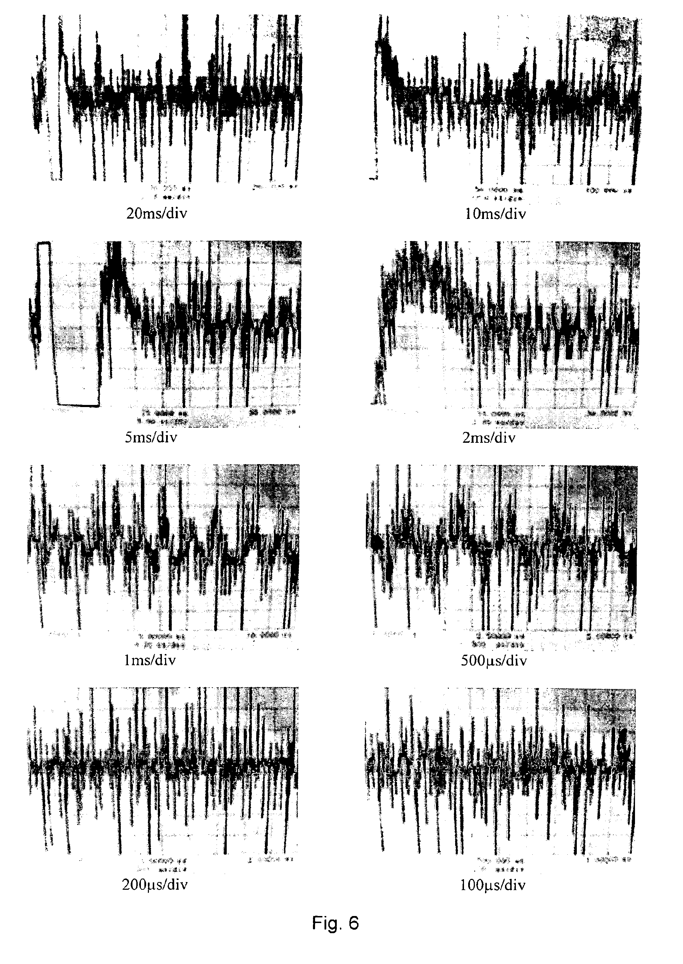

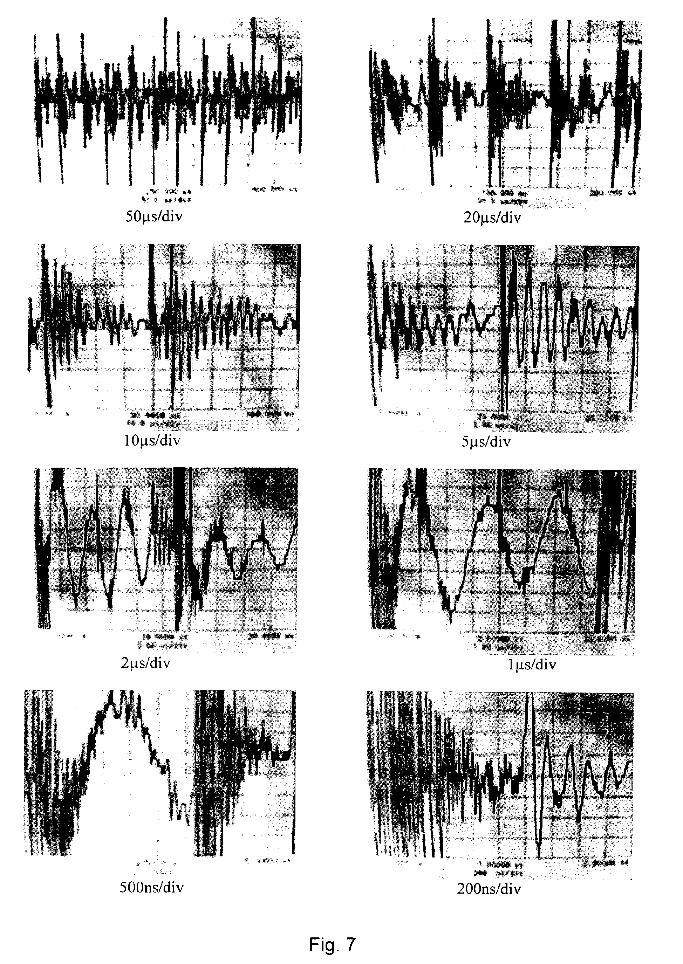

Die weitere Auflösung in den Figuren 6 und 7 zeigt, dass es sich hierbei um breitbandige sinusförmige Schwingungen mit einer Periodendauer bis in den Nanosekundenbereich handelt.

## Patentansprüche

1. Verfahren zur Erzeugung von gedämpften Entladungsströmen eines Hochspannungsenergiespeichers (3) mit überlagerten Stromimpulsen zur Beeinflussung von biologischen Zellen, **dadurch gekennzeichnet, dass** wiederholt mit Pausen der Hochspannungsenergiespeicher (3) durch Entladeströme entladen wird, die breitbandige, bis in den Nanosekundenbereich reichende Periodendauern aufweisen und anschließend aus den Entladeströmen elektromagnetische Wellen, insbesondere Longitudinalwellen generiert werden.

2. Verfahren nach Anspruch 1 **dadurch gekennzeichnet, dass** die breitbandigen Entladeströme durch das Anlegen von Hochspannungsimpulsen an eine gasgefüllte Zündkammer erzeugt werden

3. Verfahren nach Anspruch 1 oder 2, **dadurch gekennzeichnet, dass** der Hochspannungsenergiespeicher (3) maximal 3, vorzugsweise 1 mal pro Sekunde entladen wird.

4. Verfahren nach einem der vorangegangenen Ansprüche **dadurch gekennzeichnet, dass** ein Energiespeicher (3) von 70 bis 90 nF, verwendet wird.

5. Verfahren nach Anspruch 3, **dadurch gekennzeichnet, dass** eine mit einem Edelgas, vorzugsweise Xenon, gefüllte Zündkammer mit einem Überdruck im kalten Zustand von bis zu 2 bis 3 MPa verwendet wird.

6. Verfahren nach einem der vorangegangenen Ansprüche, **dadurch gekennzeichnet, dass** die Entladeströme über eine Behandlungssonde (10) mit einer Flachspule (5) und parallelen Zuleitungen (21) geführt werden.

7. Verfahren nach Anspruch 6 **dadurch gekennzeichnet, dass** eine Flachspule (5) mit 7 Windungen bei einer Kabelgesamtlänge einschließlich Zuleitungen (21) von 5,5 m ± 2 % verwendet wird.

8. Vorrichtung zur Durchführung des Verfahrens nach Anspruch 1,
**gekennzeichnet durch**
eine regelbare Gleichspannungserzeugungseinrichtung (7),
mindestens eine mit dem Ausgang der Gleichspannungserzeugungseinrichtung verbundene Hochspannungsimpulserzeugungseinrichtung (8), welche eine Hochspannung mit überlagerten Hochspannungsimpulsen generiert,
einen mittels der Hochspannung aufladbaren Energiespeicher (3) im Anschluss an die Hochspannungsimpulserzeugungseinrichtung (8),
eine gasgefüllte Zündkammer (4) zum Erzeugen eines Lichtbogens beim überschreiten der lonisationsspannung **durch** die Spannung an dem Energiespeicher (3), und
eine Behandlungssonde (10) im Anschluss an die Zündkammer (4) zur Übertragung der **durch** die Zündung ausgelösten Stromentladungsimpulse auf biologische Zellen.

9. Vorrichtung nach Anspruch 8 **dadurch gekennzeichnet, dass** die Hochspannungsimpulserzeugungseinrichtung (8) eine Hochspannungszündeinrichtung (18) mit einem ersten Hochspannungstransformator (19) aufweist, wobei die Hochspannungszündeinrichtung (18) mittels eines elektronischen Leistungsschalters (Th) gesteuert, in vorgebbaren Zeitabständen einen zweiten Hochspannungstransformator (1) speist.

10. Vorrichtung nach Anspruch 8 oder 9, **dadurch gekennzeichnet, dass** die Hochspannungszündeinrichtung Mittel (22) zum Erzeugen eines Entladestrom eines Zündladekondensators (C) und Mittel zum Erzeugen eines Induktionsstroms nach dem Entladen des Zündladekondensators (C), der durch den Entladevorgang in einem Transformator (19) aufgebauten Magnetfeld entsteht, aufweist, um eine Erhöhung der Übertragung der eingespeisten Energie zu der abgegeben Energie zu bewirken.

11. Vorrichtung nach einer der vorangegangenen Ansprüche 9 oder 10, **dadurch gekennzeichnet, dass** die der Hochspannung überlagerten Hochspannungsimpulse bis zur gleichen Höhe, vorzugsweise bis zu einer Gesamtamplitude von 30 kV aufweisen.

12. Vorrichtung nach einem der vorangegangenen Ansprüche, **dadurch gekennzeichnet, dass** die Zündkammer ein Edelgas, vorzugsweise Xenon, enthält, welches vorzugsweise unter einem Druck von bis zu 2 bis 3 MPa steht.

13. Vorrichtung nach einem der vorangegangenen Ansprüche, **dadurch gekennzeichnet, dass** die Behandlungssonde (10) eine Flachspule (5) mit parallel geführten Anschlussleitungen (21) an die Zündkammer (4) aufweist, wobei die Flachspule (5) vorzugsweise 7 Windungen bei einer Kabelgesamtlänge der Flachspule (5) mit Anschlussleitungen von 5,5 m ± 2 % aufweist.

## Geänderte Patentansprüche

### Geänderte Patentansprüche gemäss Regel 137(2) EPÜ.

**1.** Verfahren zur Erzeugung von gedämpften Entladungsströmen eines Hochspannungsenergiespeichers (3) zur Beeinflussung von biologischen Zellen, wiederholt mit Pausen der Hochspannungsenergiespeicher (3) durch Entladeströme entladen wird, **dadurch gekennzeichnet, dass**, die Entladungsströme mit Stromimpulsen überlagert werden, die breitbandige, bis in den Nanosekundenbereich reichende Periodendauern aufweisen und anschließend aus den Entladeströmen mittels einer Flachspule insbesondere Longitudinalwellen generiert werden.

**2.** Verfahren nach Anspruch 1 **dadurch gekennzeichnet, dass** die breitbandigen Entladeströme durch das Anlegen von Hochspannungsimpulsen an eine gasgefüllte Zündkammer erzeugt werden

**3.** Verfahren nach Anspruch 1 oder 2, **dadurch gekennzeichnet, dass** der Hochspannungsenergiespeicher (3) maximal 3, vorzugsweise 1 mal pro Sekunde entladen wird.

**4.** Verfahren nach einem der vorangegangenen Ansprüche **dadurch gekennzeichnet, dass** ein Energiespeicher (3) von 70 bis 90 nF, verwendet wird.

**5.** Verfahren nach Anspruch 3, **dadurch gekennzeichnet, dass** eine mit einem Edelgas, vorzugsweise Xenon, gefüllte Zündkammer mit einem Überdruck im kalten Zustand von bis zu 2 bis 3 MPa verwendet wird.

**6.** Verfahren nach einem der vorangegangenen Ansprüche, **dadurch gekennzeichnet, dass** die Entladeströme über eine Behandlungssonde (10) mit einer Flachspule (5) und parallelen Zuleitungen (21) geführt werden.

**7.** Verfahren nach Anspruch 6 **dadurch gekennzeichnet, dass** eine Flachspule (5) mit 7 Windungen bei einer Kabelgesamtlänge einschließlich Zuleitungen (21) von 5,5 m ± 2 % verwendet wird.

**8.** Vorrichtung zur Durchführung des Verfahrens nach Anspruch 1, **gekennzeichnet durch**
eine regelbare Gleichspannungserzeugungseinrichtung (7),
mindestens eine mit dem Ausgang der Gleichspannungserzeugungseinrichtung verbundene Hochspannungsimpulserzeugungseinrichtung (8), welche eine Hochspannung mit überlagerten Hochspannungsimpulsen generiert,
einen mittels der Hochspannung aufladbaren Energiespeicher (3) im Anschluss an die Hochspannungsimpulserzeugungseinrichtung (8),
eine gasgefüllte Zündkammer (4) zum Erzeugen eines Lichtbogens beim Überschreiten der Ionisationsspannung **durch** die Spannung an dem Energiespeicher (3), und
eine Behandlungssonde (10) in Form einer Flachspule (5) im Anschluss an die Zündkammer (4) zur Übertragung der **durch** die Zündung ausgelösten Stromentladungsimpulse auf biologische Zellen.

**8.** Vorrichtung nach Anspruch 8 **dadurch gekennzeichnet, dass** die Hochspannungsimpulserzeugungseinrichtung (8) eine Hochspannungszündeinrichtung (18) mit einem ersten Hochspannungstransformator (19) aufweist, wobei die Hochspannungszündeinrichtung (18) mittels eines elektronischen Leistungsschalters (Th) gesteuert, in vorgebbaren Zeitabständen einen zweiten Hochspannungstransformator (1) speist.

**10.** Vorrichtung nach Anspruch 8 oder 9, **dadurch gekennzeichnet, dass** die Hochspannungszündeinrichtung Mittel (22) zum Erzeugen eines Entladestrom eines Zündladekondensators (C) und Mittel zum Erzeugen eines Induktionsstroms nach dem Entladen des Zündladekondensators (C), der durch den Entladevorgang in einem Transformator (19) aufgebauten Magnetfeld entsteht, aufweist, um eine Erhöhung der Übertragung der eingespeisten Energie zu der abgegeben Energie zu bewirken.

**11.** Vorrichtung nach einer der vorangegangenen Ansprüche 9 oder 10, **dadurch gekennzeichnet, dass** die der Hochspannung überlagerten Hochspannungsimpulse mit einer Amplitude bis zur gleichen Höhe, vorzugsweise bis zu einer Gesamtamplitude von 30 kV aufweisen.

**12.** Vorrichtung nach einem der vorangegangenen Ansprüche, **dadurch gekennzeichnet, dass** die Zündkammer ein Edelgas, vorzugsweise Xenon, enthält, welches vorzugsweise unter einem Druck von bis zu 2 bis 3 MPa steht.

**13.** Vorrichtung nach einem der vorangegangenen Ansprüche, **dadurch gekennzeichnet, dass** die Flachspule (5) vorzugsweise 7 Windungen bei einer Kabelgesamtlänge der Flachspule (5) mit Anschlussleitungen von 5,5 m ± 2 % aufweist.
